# EUROPÄISCHE PATENTSCHRIFT

(11) **EP 1 508 560 B1**
(45) Veröffentlichungstag und Bekanntmachung des Hinweises auf die Patenterteilung: **08.10.2008**
(21) Anmeldenummer: 04028198.2
(22) Anmeldetag: 29.10.1998
(51) Int. Cl.: C07C 57/28, C07C 69/63, C07C 25/02

(54) **Zwischenprodukte zur Herstellung von substituierten Phenylketoenolen**
Intermediates for the preparation of substituted Phenylketoenols
produits intermediaires pour la preparation des phenylcetoenols substitues

(30) Priorität: 11.11.1997 DE 19749720
(43) Veröffentlichungstag der Anmeldung: 23.02.2005
(62) Teilanmeldung aus: 98956894.4
(73) Patentinhaber: Bayer CropScience AG, 40789 Monheim (DE)
(72) Erfinder: Hagemann, Hermann, Dr., 51375 Leverkusen (DE); Fischer, Reiner, Dr., 40789 Monheim (DE); Erdelen, Christoph, Dr., deceased (DE); Wachendorff-Neumann, Ulrike, Dr., 56566 Neuwied (DE); Schneider, Udo, Dr., 51373 Leverkusen (DE); Andersch, Wolfram, Dr., 51469 Bergisch Gladbach (DE)

(56) Entgegenhaltungen:
- WO-A-96/35664

## Beschreibung

Die Erfindung betrifft neue Zwischenprodükte zür Herstellung von phenylsubstituierten cyclischen Ketoenolen.

Es ist bereits bekannt geworden, daß bestimmte phenylsubstituierte cyclische Ketoenole als Insektizide, Akarizide und/oder Herbizide wirksam sind.

Bekannt sind 1H-Arylpyrrolidin-dion-Derivate (EP-A-456 063, EP-A-521 334, EP-A-596 298, EP-A-613 884, EP-A-613 885, DE-44 40 594, DE-196 49 665, WO 94/01 997, WO 95/01 358, WO 95/20 572, EP-A-668 267, WO 95/26 954, WO 96/25395, WO 96/35 664, WO 97/01 535 und WO 97/02 243) und ihre Verwendung als Schädlingsbekämpfungsmittel und zum Teil als Herbizide.

WO96/35 664 beschreibt u.a. Zwischen verbindüngen zür Herstellung von Phenylbetoenden

Die neuen Zwischenprodüktedienen zür Herstellung von Verbindungen der Formel (I) 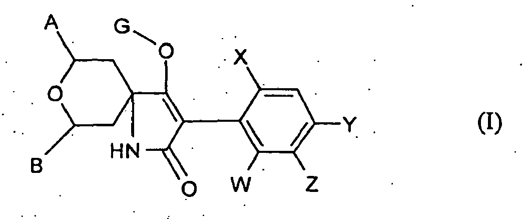 in welcher
- W: für Wasserstoff, Cyano, Nitro, Halogen, Alkyl, Alkenyl, Alkinyl, Alkoxy, Halogenalkyl, Halogenalkoxy oder für jeweils gegebenenfalls substituiertes Phenyl, Phenoxy, Phenylthio, Phenylalkoxy oder Phenylalkylthio steht,
- X: für Halogen, Alkyl, Alkenyl, Alkinyl, Alkoxy, Alkenyloxy, Halogenalkyl, Halogenalkoxy, Halogenalkenyloxy, Cyano, Nitro oder für jeweils gegebenenfalls substituiertes Phenyl, Phenoxy, Phenylthio, Phenylalkyloxy oder Phenylalkylthio steht,
- Y: für Wasserstoff, Halogen, Alkyl, Alkoxy, Halogenalkyl, Halogenalkoxy, Cyano oder Nitro steht,
- Z: für Wasserstoff, Halogen, Alkyl, Alkoxy, Halogenalkyl, Halogenalkoxy, Hydroxy, Cyano, Nitro oder für jeweils gegebenenfalls substituiertes Phenoxy, Phenylthio, 5- oder 6-gliedriges Hetaryloxy, 5- oder 6-gliedriges Hetarylthio, Phenylalkyloxy oder Phenylalkylthio steht,
- A: für Alkyl oder gegebenenfalls substituiertes Phenyl steht,
- B: für Wasserstoff oder Alkyl steht,
- G: für Wasserstoff (a) oder für einen der Reste 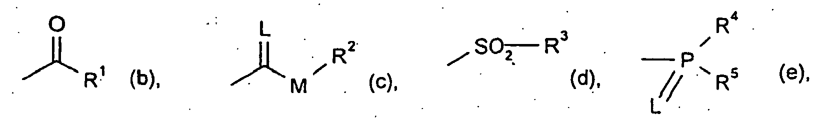 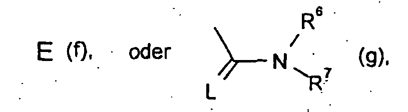 steht,
worin
E für ein Metallionäquivalent oder ein Ammoniumion steht,
L für Sauerstoff oder Schwefel steht,
M für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g) 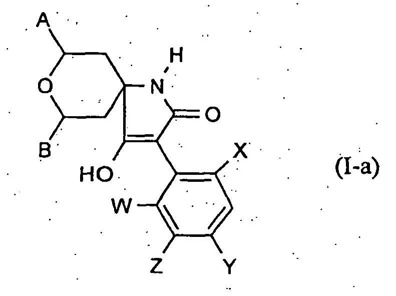 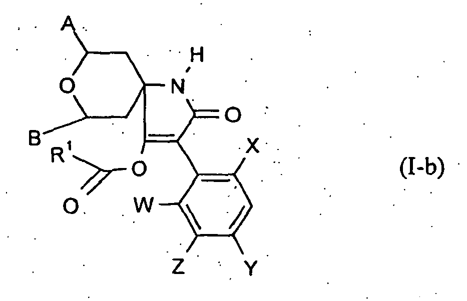 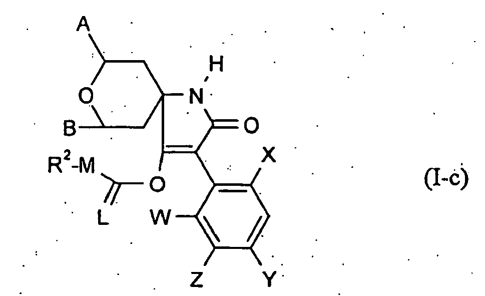 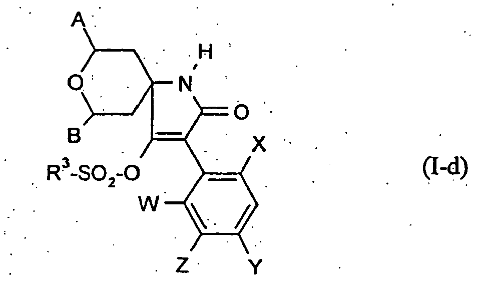 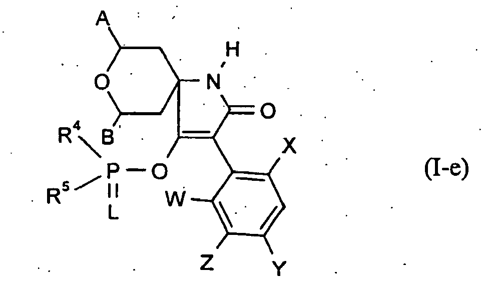 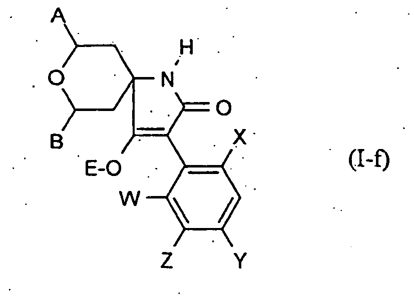 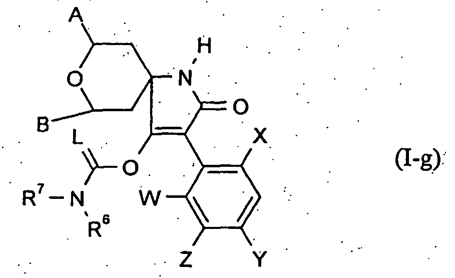 worin
A, B, E, L, M, W, X, Y, Z, R¹, R², R³, R⁴, R⁵, R⁶ und R⁷ die oben angegebenen Bedeutungen besitzen.

Die neuen Verbindungen der Formel (I) erhältman nach den im folgenden beschriebenen Verfahren :
(A) Man erhält Verbindungen der Formel (I-a) 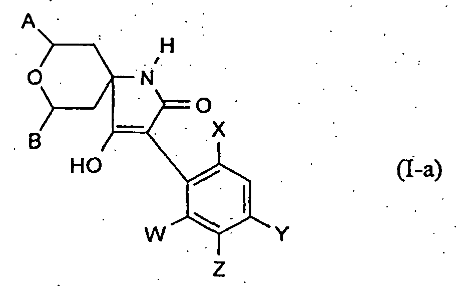 in welcher
   A, B, W, X, Y und Z die oben angegebenen Bedeutungen haben,
   wenn man
   Verbindungen der Formel (II) 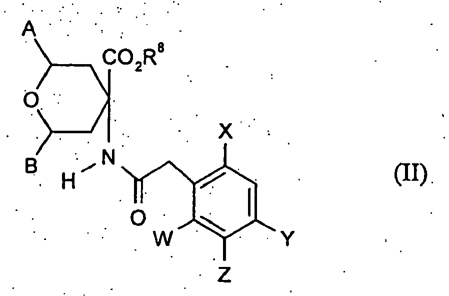 in welcher
   A, B, W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Außerdem erhält man,
(B) die Verbindungen der oben gezeigten Formel (I-b), in welcher R¹, A, B, W, X, Y und Z die oben angebenen Bedeutungen haben, wenn man Verbindungen der oben gezeigten Formel (I-a), in welcher A, B, W, X, Y und Z die oben angegebenen Bedeutungen haben,
   α) mit Säurehalogeniden der Formel (III) 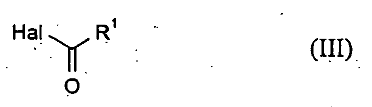 in welcher
   R¹ die oben angegebene Bedeutung hat und
   Hal für Halogen (insbesondere Chlor oder Brom) steht
   oder
   β) mit Carbonsäureanhydriden der Formel (IV)

   R¹-CO-O-CO-R¹ (IV)

   in welcher
   R¹ die oben angegebene Bedeutung hat,
   gegebenenfalls in Gegenwart eines Verdünnungsmittels und gegebenenfalls in Gegenwart eines Säurebindemittels umsetzt;
(C) Verbindungen der oben gezeigten Formel (I-c), in welcher R², A, B, W, M, X, Y und Z die oben angegebenen Bedeutungen haben und L für Sauerstoff steht, wenn man Verbindungen der oben gezeigten Formel (I-a), in welcher A, B, W, X, Y und Z die oben angegebenen Bedeutungen haben,
   mit Chlorameisensäureestern oder Chlorameisensäurethioestern der Formel (V)

   R²-M-CO-Cl (V)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(D) Verbindungen der oben gezeigten Formel (I-c), in welcher R², A, B, W, M, X, Y und Z die oben angegebenen Bedeutungen haben und L für Schwefel steht, wenn man Verbindungen der oben gezeigten Formel (I-a), in welcher A, B, W, X, Y und Z die oben angegebenen Bedeutungen haben,
   mit Chlormonothioameisensäureestern oder Chlordithioameisensäureestern der Formel (VI) 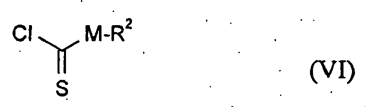 in welcher
   M und R² die oben angegebenen Bedeutungen haben,
   gegebenenfalls in Gegenwart eines Verdünnungsmittels und gegebenenfalls in Gegenwart eines Säurebindemittels umsetzt;
(E) Verbindungen der oben gezeigten Formel (I-d), in welcher R³, A, B, W, X, Y und Z die oben angegebenen Bedeutungen haben, wenn man Verbindungen der oben gezeigten Formel (I-a), in welcher A, B, W, X, Y und Z die oben angegebenen Bedeutungen haben,
   mit Sulfonsäurechloriden der Formel (VII)

   R³-SO₂-Cl (VII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(F) Verbindungen der oben gezeigten Formel (I-e), in welcher L, R⁴, R⁵, A, B, W, X, Y und Z die oben angegebenen Bedeutungen haben, wenn man Verbindungen der oben gezeigten Formel (I-a), in welcher A, B, W, X, Y und Z die oben angegebenen Bedeutungen haben,
   mit Phosphorverbindungen der Formel (VIII) 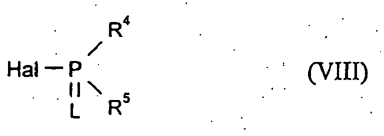 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt;
(G) Verbindungen der oben gezeigten Formeln (I-f), in welcher E, A, B, W, X, Y und Z die oben angegebenen Bedeutungen haben, wenn man Verbindungen der Formel (I-a), in welcher A, B, W, X, Y und Z die oben angegebenen Bedeutungen haben,
   mit Metallverbindungen oder Aminen der Formeln (IX) oder (X)

   Me(OR⁹)ₜ (IX)

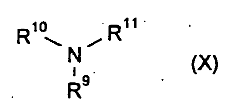

   in welchen
   Me für ein ein- oder zweiwertiges Metall (bevorzugt ein Alkali- oder Erdalkalimetall wie Lithium, Natrium, Kalium, Magnesium oder Calcium),
   t für die Zahl 1 oder 2 und
   R⁹, R¹⁰, R¹¹ unabhängig voneinander für Wasserstoff oder Alkyl (bevorzugt C₁-C₈-Alkyl) stehen,
   gegebenenfalls in Gegenwart eines Verdünnungsmittels umsetzt;
(H) Verbindungen der oben gezeigten Formel (I-g), in welcher L, R⁶, R⁷, A, B, W, X, Y und Z die oben angegebenen Bedeutungen haben, wenn man Verbindungen der oben gezeigten Formel (I-a), in welcher A, B, W, X, Y und Z die oben angegebenen Bedeutungen haben,
   α) mit Isocyanaten oder Isothiocyanaten der Formel (XI)

   R⁶-N=C=L (XI)

   in welcher
   R⁶ und L die oben angegebenen Bedeutungen haben,
   gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XII) 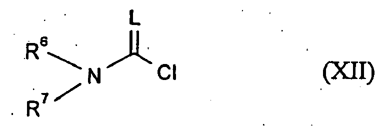 in welcher
   L, R⁶ und R⁷ die oben angegebenen Bedeutungen haben,
   gegebenenfalls in Gegenwart eines Verdünnungsmittels und gegebenenfalls in Gegenwart eines Säurebindemittels, umsetzt.

Verbindungen der Formel (I) weisen eine sehr gute Wirksamkeit als Schädlingsbekämpfungsmittel, vorzugsweise als Insektizide und Akarizide und als Herbizide auf und sind darüber hinaus häufig sehr gut pflanzenverträglich, insbesondere gegenüber Kulturpflanzen.

Die beim erfindungsgemäßen Verfahren (A) als Ausgangsstoffe benötigten Verbindungen der Formel (II) 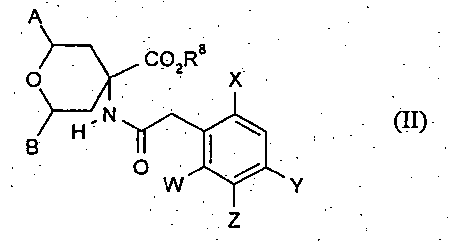 in welcher
A, B, W, X, Y, Z und R⁸ die oben angegebenen Bedeutungen haben,
sind neu, alies nich gegenstend dieser Erfindung.

Man erhält die Acylaminosäureester der Formel (II) beispielsweise, wenn man Aminosäurederivate der Formel (XIII) 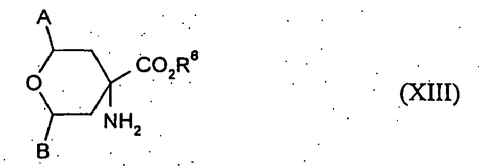 in welcher
A, B und R⁸ die oben angegebene Bedeutung haben,
mit substituierten Phenylessigsäurehalogeniden der Formel (XIV) 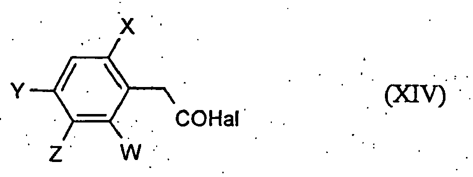 in welcher
W, X, Y und Z die oben angegebenen Bedeutungen haben und
Hal für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XV) 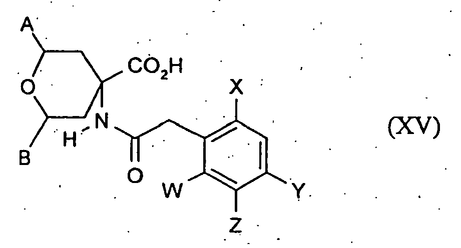 in welcher
A, B, W, X, Y und Z die oben angegebenen Bedeutungen haben,
verestert (Chem. Ind. (London) 1568 (1968)).

Die Verbindungen der Formel (XV) 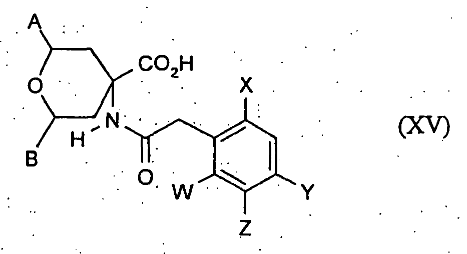 in welcher
A, B, W, X, Y und Z die oben angegebenen Bedeutungen haben,
sind neu, aber wich gegewofand dieser Erfindung

Man erhält die Verbindungen der Formel (XV) beispielsweise, wenn man 4-Amino-tetrahydropyran-4-carbonsäuren der Formel (XVI) 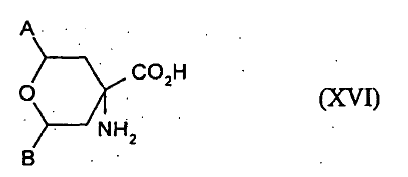 in welcher
A und B die oben genannten Bedeutungen haben,
mit substituierten Phenylessigsäurehalogeniden der Formel (XIV) 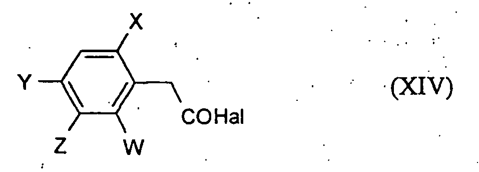 in welcher
W, X, Y und Z die oben angegebenen Bedeutungen haben und
Hal für Chlor oder Brom steht,
nach Schotten-Baumann acyliert (Organikum, VEB Deutscher Verlag der Wissenschaften, Berlin 1977, S. 505).

Die Verbindungen der Formel (XIV) sind teilweise neu und lassen sich nach bekannten Verfahren herstellen (vgl. z.B. DE-196 49 665).

Man erhält die Verbindungen der Formel (XIV) beispielsweise, indem man substituierte Phenylessigsäuren der Formel (XVII) 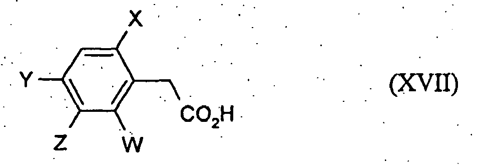 in welcher
W, X, Y und Z die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XVII) sind teilweise neu, sie lassen sich nach literaturbekannten Verfahren herstellen (Organikum 15. Auflage, S. 533, VEB Deutscher Verlag der Wissenschaften, Berlin 1977, vgl. z.B. DE-196 49 665).

Man erhält die Verbindungen der Formel (XVII) beispielsweise, indem man substituierte Phenylessigsäureester der Formel (XVIII) 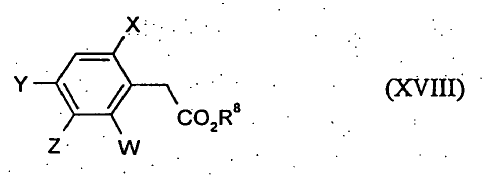 in welcher
W, X, Y, Z und R⁸ die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XVIII) sind teilweise neu, sie lassen sich nach im Prinzip bekannten Verfahren herstellen.

Man erhält die Verbindungen der Formel (XVIII) beispielsweise, indem man substituierte 1,1,1 -Trichlor-2-phenylethane der Formel (XIX) 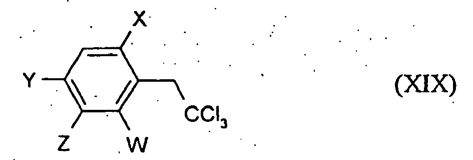 in welcher
W, X, Y und Z die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE-3 314 249).

Die Verbindungen der Formel (XIX) sind teilweise neu, sie lassen sich nach im Prinzip bekannten Verfahren herstellen.

Man erhält die Verbindungen der Formel (XIX) beispielsweise, wenn man Aniline der Formel (XX) 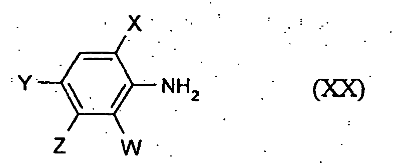 in welcher
W, X, Y und Z die oben angegebene Bedeutung haben,
in Gegenwart eines Alkylnitrits der Formel (XXI)

R¹³-ONO (XXI)

in welcher
R¹³ für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XX) sind teilweise bekannt. Sie lassen sich nach literaturbekannten Verfahren darstellen, beispielsweise durch Reduktion der entsprechenden Nitroverbindungen oder Halogenierung der Aniline bzw. Acetanilide und anschließende Rückspaltung.

Die Verbindungen der Formel (XXI) sind bekannte Verbindungen der Organischen Chemie. Kupfer(II)-chlorid und Vinylidenchlorid sind lange bekannt und käuflich.

Die substituierten cyclischen Aminocarbonsäuren der Formel (XVI) sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

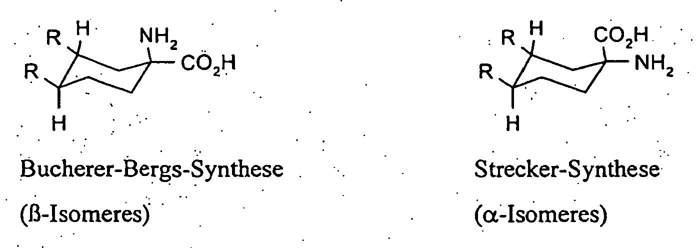

Die Verbindungen der Formel (XIII) und (XVI) sind neu. Sie lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975)).

Weiterhin lassen sich die bei dem obigen Verfahren (A) verwendeten Ausgangsstoffe der Formel (II) 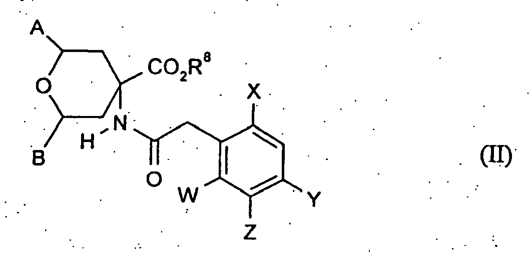 in welcher
A, B, W, X, Y, Z und R⁸ die oben angegebenen Bedeutungen haben,
herstellen, wenn man Aminonitrile der Formel (XXII) 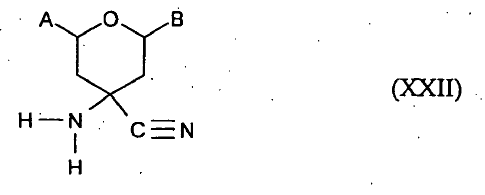 in welcher
A und B die oben angegebenen Bedeutungen haben,
mit substituierten Phenylessigsäurehalogeniden der Formel (XIV) 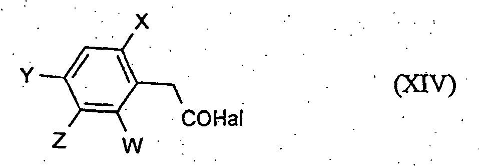 in welcher
W, X, Y, Z und Hal die oben angegebenen Bedeutungen haben,
zu Verbindungen der Formel (XXIII) 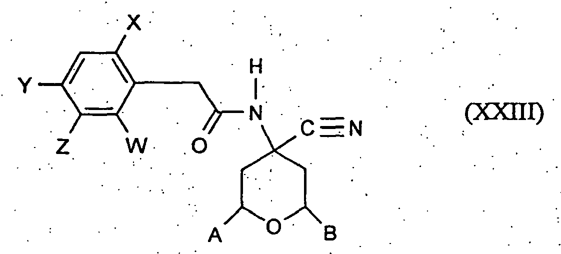 in welcher
A, B, W, X, Y und Z die oben angegebenen Bedeutungen haben,
umsetzt,
und diese anschließend einer sauren Alkoholyse unterwirft.

Die Verbindungen der Formel (XXIII) sind ebenfalls neu. Die Verbindungen der Formel (XXII) sind ebenfalls neu.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) sind allgemein bekannte Verbindungen der organischen bzw. anorganischen Chemie.

Die Verbindungen der Formeln (XIV), (XVII), (XVIII), (XIX) und (XX) sind darüber hinaus aus den eingangs zitierten Patentanmeldungen bekannt und/oder lassen sich nach den dort angegebenen Methoden herstellen (vgl. auch DE-A 196 49 665 und DE-A 196 13 171).

Das Verfahren (A) ist dadurch gekennzeichnet, daß man Verbindungen der Formel (II), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und - carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B_{α}) ist dadurch gekennzeichnet, daß man Verbindungen der Formel (I-a) mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (B_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (B_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (B_{α}) werden die Ausgangsstoffe der Formel (I-a) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (I-a) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Bβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (I-a) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (I-a) mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (C) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (VII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (D) ist dadurch gekennzeichnet, daß man Verbindungen der Formel (I-a) mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen der Formel (I-a) dar, kann auf den weiteren Zusatz von Säurebindemitteln verzichtet werden.

Als Basen können beim Verfahren (D) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, daß man Verbindungen der Formel (I-a) jeweils mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (IX) bei -20 bis 150°C, vorzugsweise bei 0 bis 70°C um.

Das Verfahren (E) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, daß man Verbindungen der Formeln (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen der Formel (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Das Verfahren (F) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (G) ist dadurch gekennzeichnet, daß man Verbindungen der Formel (I-a) jeweils mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (H) ist dadurch gekennzeichnet, daß man Verbindungen der Formel (I-a) jeweils mit (Hα) Verbindungen der Formel (XI) gegebenenfalls in Gegenwart eines Verdünnungsmittels und gegebenenfalls in Gegenwart eines Katalysators oder (Hβ) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (Hα) setzt man pro Mol Ausgangsverbindung der Formel (I-a) ca. 1 Mol Isocyanat der Formel (XI) bei 0 bis 100°C, vorzugsweise bei 20 bis 50°C um.

Das Verfahren (Hα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Hβ) setzt man pro Mol Ausgangsverbindung der Formel (I-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe des Formel (I) eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Gegenstand dieser Erfindung sind Verbindungen der Formel (XVII) 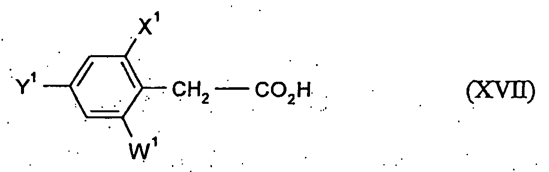 in welcher
X¹ für Fluor, Chlor oder Brom steht,
Y¹ für Ethyl steht und
W¹ für Wasserstoff, Fluor, Chlor oder Brom steht,
X¹ bevorzügt für Chlor oder Brom steht,
Y¹ bevozugt für Ethyl steht und
W¹ bevorzugt für Wasserstoff, Chlor oder Brom steht.

Gegenstand dieses Erfindung sind auch Verbindungen der Formel (XVIII) 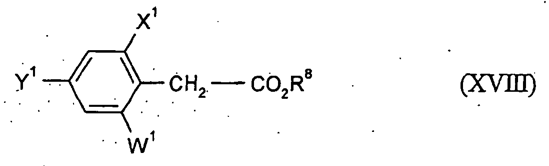 in welcher
X¹, Y¹ und W¹ die angegebenen Bedeutungen haben und
R⁸ für Alkyl steht, bevorzugt für C₁-C₆-Alkyl steht, besonders bevorzügt für Methyl oder Ethyl steht,

Gegenstand dieser Erfindung sind ebenfalls Verbindungen der Formel (XIX) 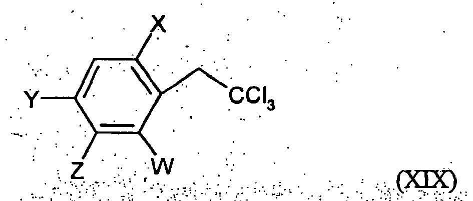 in welcher
X für X¹ steht,
Y für Y¹ steht,
W für W⁻¹ steht und
Z für wasserstoff steht.

Die Herstellung der neuien Zwischenprodükte geht aus den nachforgenden Beispielen hervor.

### Herstellungsbeispiele

### Beispiel (XVII-1)

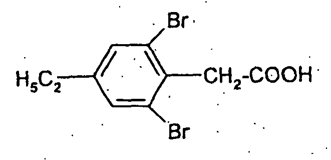

102,5 g (16,9 %ig, 0,05 mol) der Verbindung gemäß Beispiel (XVIII-1), 14,1 g KOH, 17,8 ml Wasser und 35,5 ml Methanol werden zusammen 5 Stunden unter Rückfluß erhitzt. Dann engt man ein und nimmt den Rückstand in Wasser auf. Man wäscht mit Essigester und säuert die wäßrige Phase mit konz. HCl an (pH 1). Man saugt ab und trocknet.
Ausbeute 14,4 g (80,6 % der Theorie), Fp.: 140-142°C.
Analog bzw. gemäß den allgemeinen Angaben zur Herstellung erhält man folgende Verbindungen der Formel (XVII):

**Tabelle 7**

| | | | | | |
|---|---|---|---|---|---|
| 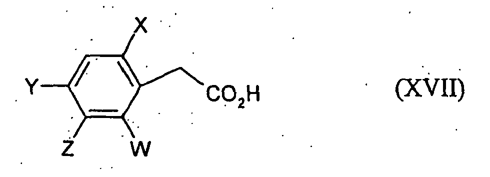 | | | | | |

| Bsp.-Nr. | W | X | Y | Z | Fp.°C |
|---|---|---|---|---|---|
| XVII-2 | Br | Cl | C₂H₅ | H | 147 |
| XVII-3 | Cl | Cl | C₂H₅ | H | 146 |
| XVII-4 | H | Cl | C₂H₅ | H | 89-91 |
| XVII-5 | H | Br | C₂H₅ | H | 109 |
| XVII-6 | Br | Br | i-C₃H₇ | H | 154-155 |
| XVII-7 | H | CH₃ | Cl | Cl | 103 |

### Beispiel (XVIII-1)

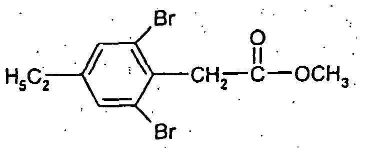

Zu 5 g (94,4 %ig, 0,0119 mol) der Verbindung gemäß Beispiel (XIX-1) in 5 ml Methanol tropft man unter Kühlung 9,1 ml 30 %iges Natriummethylat und rührt 5 Stunden unter Rückfluß. Nach dem Abkühlen tropft man 0,01 ml konzentrierte Schwefelsäure zu und rührt 1 Stunde unter Rückfluß. Dann wird eingeengt und der Rückstand in Wasser aufgenommen. Man extrahiert mit Methylenchlorid, trocknet und engt ein.
Ausbeute 1,80 g (43 % der Theorie), Öl.
Analog bzw. gemäß den allgemeinen Angaben zur Herstellung erhält man folgende Verbindungen der Formel (XVIII):

**Tabelle 8**

| | | | | | | | |
|---|---|---|---|---|---|---|---|
| 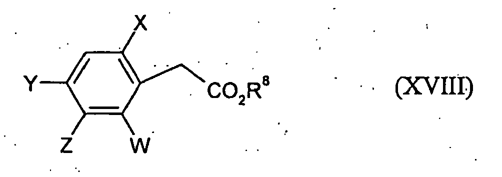 | | | | | | | |

| Bsp.-Nr. | W | X | Y | Z | R⁸ | Kp°C | (mbar) |
|---|---|---|---|---|---|---|---|
| XVIII-2 | Br | Cl | C₂H₅ | H | CH₃ | 105 | 0.06 |
| XVIII-3 | Cl | Cl | C₂H₅ | H | CH₃ | 92-94 | 0.05 |
| XVIII-4 | H | Cl | C₂H₅ | H | CH₃ | 82 | 0.03 |
| XVIII-5 | H | Br | C₂H₅ | H | CH₃ | 135 | 0.15 |
| XVIII-6 | Br | Br | i-C₃H₇ | H | CH₃ | Öl* | |
| XVIII-7 | H | CH₃ | Cl | Cl | CH₃ | Öl* | |

| | | | | | | | |
|---|---|---|---|---|---|---|---|
| * Nach chromatographischer Reinigung wurden diese Verbindungen direkt zu den entsprechenden Säuren der Formeln (XVII-6) und (XVII-7) umgesetzt. | | | | | | | |

### Beispiel (XIX-1)

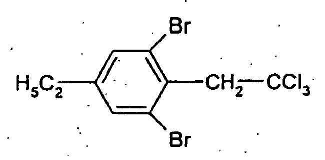

Zu 208 ml (1,746 mol) Butylnitrit in 684 ml wasserfreiem Acetonitril tropft man 1400 ml (17,4 mol) 1,1-Dichlorethan und dann 320 g (1,147 mol) der Verbindung gemäß Beispiel (XX-1) in 342 ml wasserfreiem Acetonitril. Man rührt über Nacht bei Raumtemperatur und gießt dann auf 4,61 20 %ige HCl. Man extrahiert mit MTB-Ether, wäscht die organische Phase mit 21 Wasser, trocknet und engt ein.
Ausbeute 434 g. Das Rohprodukt wird ohne weitere Reinigung weiter umgesetzt.

Analog bzw. gemäß den allgemeinen Angaben zur Herstellung erhält man folgende Verbindungen der Formel (XIX):

**Tabelle 9**

| | | | | | |
|---|---|---|---|---|---|
| 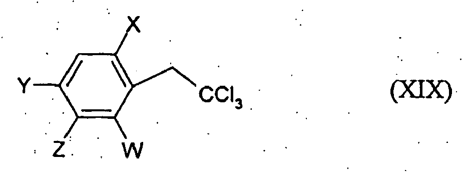 | | | | | |

| Bsp.-Nr. | W | X | Y | Z | |
|---|---|---|---|---|---|
| XIX-2 | Br | Cl | C₂H₅ | H | Öl* |
| XIX-3 | Cl | Cl | C₂H₅ | H | Öl* |
| XIX-4 | H | Cl | C₂H₅ | H | Öl* |
| XIX-5 | H | Br | C₂H₅ | H | GC/MS: |
| | | | | | 314, 316, 318 |
| | | | | | 12%, 14%, 12% |
| | | | | | 199 (100 %) |
| | | | | | 197 (98 %) |
| XIX-6 | Br | Br | i-C₃H₇ | H | Öl* |
| XIX-7 | H | CH₃ | Cl | Cl | Öl* |

| | | | | | |
|---|---|---|---|---|---|
| * Die Rohgemische wurden direkt in die Alkoholyse zur Herstellung der Verbindungen der Formel (XVIII) eingesetzt. | | | | | |

### Beispiel (XX-1)

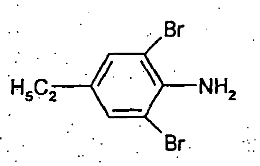

Zu 150 g (1,24 mol) 4-Ethylanilin in 1990 ml Eisessig tropft man bei 10-30°C 397 g (2,48 mol) Brom in 744 ml Eisessig und rührt noch 3 Stunden bei 30°C. Dann verdünnt man mit Wasser und stellt mit 25 %iger Ammoniaklösung alkalisch. Man saugt ab, nimmt in Methylenchlorid auf, trocknet und engt ein.

Ausbeute 320,0 g (93 % der Theorie), Fp.: 74°C.

### Beispiel (XX-2)

Analog zu Beispiel (XX-1) erhält man die Verbindung der Formel 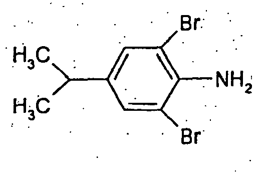 Fp.: 48°C.

### Beispiel (XXII-1)

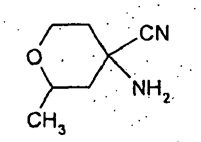

Zum Gemisch bestehend aus 50,9 g (0,75 mol) 25 %iger Ammoniaklösung, 17,2 g (0,32 mol) Ammoniumchlorid und 15,7 g (0,32 mol) Natriumcyanid in 48 ml Wasser tropft man bei Raumtemperatur 30,5 g (0,27 mol) 2-Methyl-tetrahydropyran-4-on (Herstellung siehe weiter unten) und rührt über Nacht bei 45°C. Nach üblicher Aufarbeitung erhält man 29,1 g (77 % der Theorie) Endprodukt als Öl.

### Beispiel (XXII-2)

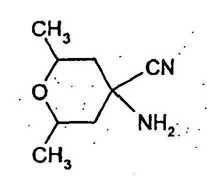

Diese Verbindung erhält man in analoger Weise als braunes Öl.

### Beispiel: Verbindung der Formel

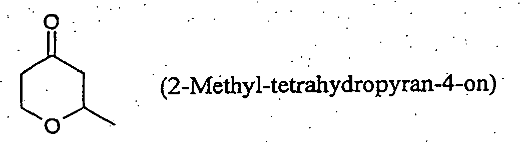

Zu 552,72 g (3,54 mol) NaH₂PO₄ x 2 H₂O und 179,7 g (1,55 mol) 85 %iger o-Phosphorsäure in 5500 ml Wasser tropft man bei einer Temperatur von ca. 100°C innerhalb von ca. 75 Minuten 364,37 g der Verbindung mit der Formel ClCH₂CH₂COCH₂CHClCH₃ (Herstellung siehe folgendes Beispiel) und rührt noch 8 Stunden bei 100°C.

Es wird auf ca. 0°C abgekühlt und 10 molare NaOH zugetropft, bis ein pH-Wert von 5 bis 6 erreicht ist. Nach Zugabe von 1500 ml Methylenchlorid wird das entstandene Salz abgesaugt und die wäßrige Phase 3 x mit je 1000 ml Methylenchlorid extrahiert. Man trocknet, engt ein und destilliert.

Ausbeute: 119,6 g (55 % der Theorie), Kp.: 62°C/15 mbar.

### Beispiel: Verbindung der Formel ClCH₂CH₂COCH₂CHClCH₃

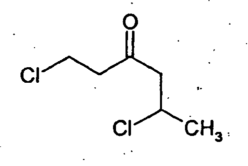

Zu 758,08 g (5,6 mol) AlCl₃ in 560 ml Methylenchlorid tropft man bei Raumtemperatur innerhalb von 15 Minuten 507,88 g 3-Chlorpropionylchlorid und leitet bei ca. 28 bis 30°C innerhalb von ca. 3 Stunden 189 g (4,5 mol) Propylen ein.

Vom überschüssigen AlCl₃ wird abdekantiert und das Reaktionsgemisch bei 0 bis 10°C langsam in ein Gemisch aus 508 ml Methylenchlorid und 2032 ml 1 N HCl getropft.

Die organische Phase wird abgetrennt, 3 mal mit je 500 ml Wasser gewaschen, getrocknet und eingeengt.

Ausbeute: 470 g (70 % der Theorie).

## Patentansprüche

1. Verbindungen der Formel (XVII) 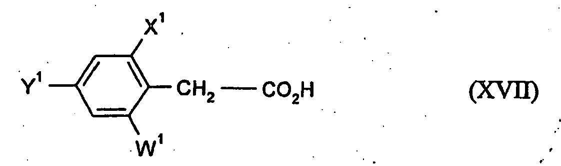 in welcher
X¹ für Fluor, Chlor oder Brom steht,
Y¹ für Ethyl steht und
W¹ für Wasserstoff, Fluor, Chlor oder Brom steht.

2. Verbindungen der Formel (XVII) gemäß Anspruch 1, in welcher
X¹ für Chlor oder Brom steht,
Y¹ für Ethyl steht und
W¹ für Wasserstoff, Chlor oder Brom steht.

3. Verbindungen der Formel (XVIII) 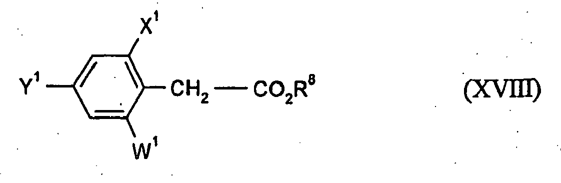 in welcher
X¹, Y¹ und W¹ die in Anspruch 1 oder 2 angegebenen Bedeutungen haben und
R⁸ für Alkyl steht.

4. Verbindungen der Formel (XVIII) gemäß Anspruch 3, in welcher
R⁸ für C₁-C₆-Alkyl steht.

5. Verbindungen der Formel (XVIII) gemäß Anspruch 3, in welcher
R⁸ für Methyl oder Ethyl steht.

6. Verbindungen der Formel (XIX) 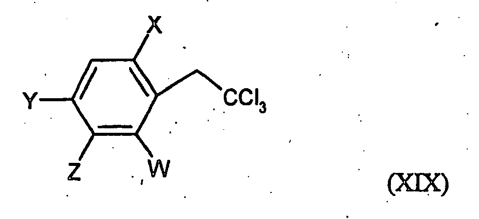 in welcher
X für X¹ steht,
Y für Y¹ steht,
W für W¹ steht und
Z für Wassestoff steht.

7. Verbindungen der Formel (XIX) gemäß Anspruch 6, in welcher W, X, Y und Z die in der Tabelle angegebenen Bedeutungen haben:
| W | X | Y | Z | |
|---|---|---|---|---|
| Br | Cl | C₂H₅ | H | (Bsp. XIX-2) |
| Cl | Cl | C₂H₅ | H | (Bsp. XIX-3) |
| Br | Br | C₂H₅ | H | (Bsp. XIX-1) |

## Claims

1. Compounds of the formula (XVII) 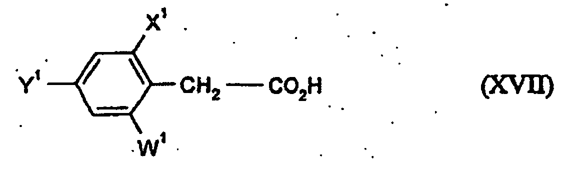 in which
X¹ represents fluorine, chlorine or bromine,
Y¹ represents ethyl and
W¹ represents hydrogen, fluorine, chlorine or bromine.

2. Compounds of the formula (XVII) according to Claim 1, in which
X¹ represents chlorine or bromine,
Y¹ represents ethyl and
W¹ represents hydrogen, chlorine or bromine.

3. Compounds of the formula (XVIII) 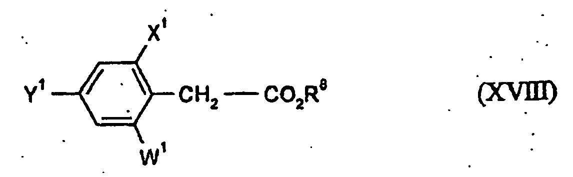 in which
X¹, Y¹ and W¹ have the meanings given in Claim 1 or 2 and
R⁸ represents alkyl.

4. Compounds of the formula (XVIII) according to Claim 3, in which
R⁸ represents C₁-C₆-alkyl.

5. Compounds of the formula (XVIII) according to Claim 3, in which
R⁸ represents methyl or ethyl.

6. Compounds of the formula (XIX) 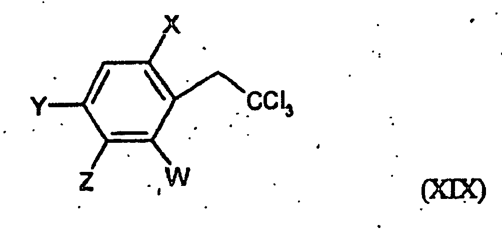 in which
X represents X¹,
Y represents Y¹,
W represents W¹ and
Z represents hydrogen.

7. Compounds of the formula (XIX) according to Claim 6, in which W, X, Y and Z have the meanings given in the table:
| W | X | Y | Z | |
|---|---|---|---|---|
| Br | Cl | C₂H₅ | H | (Ex. XIX-2) |
| Cl | Cl | C₂H₅ | H | (Ex. XIX-3) |
| Br | Br | C₂H₅ | H | (Ex. XIX-1) |

## Revendications

1. Composés de formule (XVII) 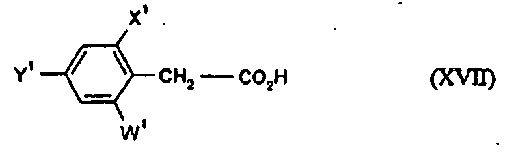 dans laquelle
X¹ représente le fluor, le chlore ou le brome,
Y¹ est un reste éthyle et
W¹ représente l'hydrogène, le fluor, le chlore ou le brome.

2. Composés de formule (XVII) suivant la revendication 1, formule dans laquelle
X¹ représente le chlore ou le brome,
Y¹ est un reste éthyle et
W¹ représente l'hydrogène, le chlore ou le brome.

3. Composés de formule (XVIII) 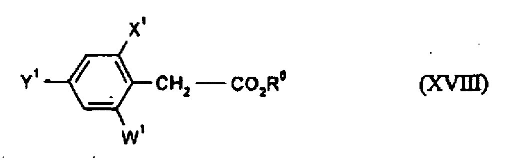 dans laquelle
X¹, Y¹ et W¹ ont les définitions indiquées dans la revendication 1 ou 2 et
R⁸ est un reste alkyle.

4. Composés de formule (XVIII) suivant la revendication 3, formule dans laquelle
R⁸ est un reste alkyle en C₁ à C₆.

5. Composés de formule (XVIII) suivant la revendication 3, formule dans laquelle
R⁸ est un reste méthyle ou éthyle.

6. Composés de formule (XIX) 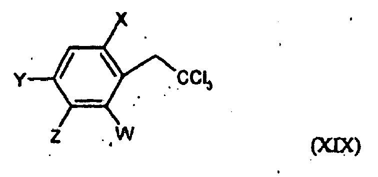 dans laquelle
X représente X¹,
Y représente Y¹,
W représente W¹ et
Z représente l'hydrogène.

7. Composés de formule (XIX) suivant la revendication 6, formule dans laquelle W, X, Y et Z ont les définitions indiquées dans le tableau :
| W | X | Y | z | |
|---|---|---|---|---|
| Br | Cl | C₂H₅ | H | (Ex. XIX-2) |
| Cl | Cl | C₂H₅ | H | (Ex. XIX-3) |
| Br | Br | C₂H₅ | H | (Ex. XIX-1) |
